(19) Europäisches Patentamt
European Patent Office
Office européen des brevets

(11) **EP 4 041 170 B1**

(12) **EUROPEAN PATENT SPECIFICATION**

(45) Date of publication and mention
of the grant of the patent:
**19.11.2025 Bulletin 2025/47**

(21) Application number: **20873505.0**

(22) Date of filing: **12.06.2020**

(51) International Patent Classification (IPC):
*A61F 13/15* (2006.01)      *A61F 13/49* (2006.01)
*A61F 13/496* (2006.01)     *A61F 13/512* (2006.01)
*A61F 13/514* (2006.01)

(52) Cooperative Patent Classification (CPC):
**A61F 13/49011; A61F 13/15593; A61F 13/4902;
A61F 13/4963; A61F 13/512; A61F 13/5146**

(86) International application number:
**PCT/CN2020/095763**

(87) International publication number:
**WO 2021/068544 (15.04.2021 Gazette 2021/15)**

(54) **RING-LIKE ELASTIC BELT AND METHOD OF MAKING THEREOF**

**RINGARTIGES ELASTISCHES BAND UND VERFAHREN ZU SEINER HERSTELLUNG**

**COURROIE ÉLASTIQUE ANNULAIRE ET SA MÉTHODE DE FABRICATION**

(84) Designated Contracting States:
**AL AT BE BG CH CY CZ DE DK EE ES FI FR GB
GR HR HU IE IS IT LI LT LU LV MC MK MT NL NO
PL PT RO RS SE SI SK SM TR**

(30) Priority: **08.10.2019 PCT/CN2019/109917**

(43) Date of publication of application:
**17.08.2022 Bulletin 2022/33**

(73) Proprietor: **The Procter & Gamble Company
Cincinnati, OH 45202 (US)**

(72) Inventors:
• **LEE, DonSub**
 **Hyogo 6740093 (JP)**
• **TSUNEKI, Seiji**
 **Hyogo 6740093 (JP)**
• **MORIMOTO, Koichi**
 **Beijing 101300 (CN)**
• **QUADE, Michael Brian**
 **Cincinnati, Ohio 45202 (US)**
• **HUANG, Gene Xiaoqing**
 **Cincinnati, Ohio 45202 (US)**

(74) Representative: **P&G Patent Germany
Procter & Gamble Service GmbH
Sulzbacher Straße 40
65824 Schwalbach am Taunus (DE)**

(56) References cited:
WO-A1-2016/029374     WO-A1-2016/029374
WO-A1-2017/133031     WO-A1-2017/133031
WO-A1-2018/152833     WO-A1-2018/152833
CN-A- 105 982 789     CN-A- 106 659 611
CN-A- 107 106 357     CN-A- 108 175 579
CN-A- 108 175 579     JP-A- 2002 065 733
JP-A- 2008 245 884     JP-A- 2017 029 636
JP-A- 2017 029 636     US-A1- 2013 261 589
US-A1- 2013 261 589     US-A1- 2018 221 218
US-A1- 2018 221 218

**Description**

FIELD OF THE INVENTION

The present invention relates to a ring-like elastic belt for a wearable article, and method of making thereof.

BACKGROUND OF THE INVENTION

Absorbent articles such as diapers have areas of elasticity to provide a snug fit on the wearer. Such areas of elasticity are provided by elastic members to be used as waist bands and leg bands. In some configurations, it may be advantageous for certain areas of the elastic members to be free of elasticity. For example, it may not be desirous to have elasticity in the front and rear section of the diaper matching the area where an absorbent core overlaps. If elasticity is present in the absorbent core overlapping area, this may be uncomfortable for the wearer, and also cause unnecessary bunching up of material deteriorating the absorbing efficiency of the absorbent core. Furthermore, absorbent articles, especially baby diapers, are commonly designed with artwork on the front and/or rear section of the diaper. Such artwork may provide a connotation to quality of the product, amusement to the wearer and caregiver, and may also be used for educational or training purposes. Absence of elasticity in those sections helps prevent the artwork to be distorted. Meanwhile, it is often desirable to have such elastic member cutting conducted in a cost efficient manner with minimum influence to production speed such as those processes described in, but not limited to, US7867529, US9039855, US9050213, US9028632, US8440043, US9364965, and US9738002.

In recent years, various nonwoven materials have been suggested and used for making the garment-facing surface of the absorbent article to have certain functionality, such as softness, loftiness and breathability. To provide such functionality, the nonwoven materials may be provided in higher caliper or basis weight, which may require special or additional handling needs during manufacture. Further, consciousness of skin health of the wearer has increased, that forces on the elastic belt are desired to be as low as reasonably required for keeping the article from sagging. On the other hand, in that nonwoven materials having functionality and accurately configured elastic belts may be used for products of higher consumer value, quality assurance of such absorbent article must also have a high standard.

US2018221218A1 relates to a wearable article continuous in a longitudinal direction and a transverse direction, comprising a main body and an elastic belt comprising a front belt and a back belt. The transverse edges of the front belt and the back belt are joined by a seam to form a waist opening and two leg openings. Each of the front belt and back belt are formed by an inner sheet, an outer sheet, and a plurality of elastic bodies sandwiched therebetween. At least some of the elastic bodies run in the transverse direction substantially parallel to each other. The articles exhibit a Belt Utilization of no less than 92%, and a Fit Circumference Force of greater than 2.0N.

WO2018152833A1 in concerned with a wearable article continuous in a longitudinal direction and a transverse direction comprising an elastic belt region, a crotch region, a waist opening and two leg openings. The elastic belt region is a laminate comprising an inner sheet made, and an outer sheet, each made of nonwoven fiber, and a plurality of elastic bodies. The wearable article exhibits a Compression Work index of at least about 70gfcm and a Stretch Circumference Force of less than about 6.5N.

WO2017133031A1 discloses a wearable article comprising a main body and a ring-like elastic belt, again with a front belt and a back belt. The entirety of the length of the front belt side edge is seamed with the back belt side edge. The front and back belts are each divided into 4 zones. For the front belt, the tensile stress of the proximal tummy zone is higher than the tensile stress of any of the waist zone, the distal tummy zone, or the leg zone. For the back the tensile stress of the distal tummy zone is higher than in the three other zones.

WO2016029374A1 relates to a wearable article comprising a main body and a ring-like elastic belt comprising a front belt and a back belt. At least some of the elastic bodies are arranged in an array, which is formed by 2-10 elastic bodies. Each elastic body within one array is disposed in an inner-interval between each elastic body of 2-4 mm. One specific array disposed in an extra-interval between at least one neighboring elastic body outside the specific array of greater than the inner-interval. The back belt (86) comprises at least 5 arrays.

US2013261589A1 in concerned with apparatuses for cutting an elastic member of absorbent articles. The cutting roll system includes: an anvil roll; and a cutting roll adjacent the anvil roll, which is adapted to rotate about a roll axis. The cutting roll comprises a knife block; which comprises at least two rows of knife edges, which each have two or more knife edges arrayed linearly parallel to the roll axis and are circumferentially spaced apart from each other. All knife edges are oriented at an angle which does not match with the roll axis direction or a perpendicular direction.

Based on the foregoing, there remains a need for providing a reliable elastic member cutting system which can be used over a variety of materials used for the absorbent article, which is cost efficient, and which has little influence on production speed.

SUMMARY OF THE INVENTION

The present invention provides a ring-like elastic belt according to the claims , a wearable article comprising the aforementioned ring-like elastic belt a method of making the aforementioned ring-like elastic belt.

BRIEF DESCRIPTION OF THE DRAWINGS

While the specification concludes with claims particularly pointing out and distinctly claiming the subject matter which is regarded as forming the present invention, it is believed that the invention will be better understood from the following description which is taken in conjunction with the accompanying drawings and which like designations are used to designate substantially identical elements, and in which:

Figure 1 is a perspective view of one embodiment of a wearable article of the present invention.

Figure 2 is a schematic plan view of one embodiment of a wearable article of the present invention with the seams unjoined and removed, and in a flat uncontracted condition showing the garment facing surface.

Figure 3A is a cross section view of Figure 2 taken along L1.

Figure 3B is a cross section view of Figure 2 taken along L2.

Figure 4 is a schematic view of the process of the present invention.

Figure 5 is a cross section of the cutting roll system of the present invention.

Figure 6 is a schematic view of the blade of the present invention.

Figure 7 is a schematic view of an example of a hanger-type sample holding fixture according to the "Whole Article Force Measurement".

DEFINITIONS

As used herein, the following terms shall have the meaning specified thereafter:
"Wearable article" refers to articles of wear which may be in the form of pants, taped diapers, incontinent briefs, feminine hygiene garments, and the like. The "wearable article" may be so configured to also absorb and contain various exudates such as urine, feces, and menses discharged from the body. The "wearable article" may serve as an outer cover adaptable to be joined with a separable disposable absorbent insert for providing absorbent and containment function, such as those disclosed in PCT publication WO 2011/087503A.
"Pant" refers to disposable absorbent articles having a pre-formed waist and leg openings. A pant may be donned by inserting a wearer's legs into the leg openings and sliding the pant into position about the wearer's lower torso. Pants are also commonly referred to as "closed diapers", "prefastened diapers", "pull-on diapers", "training pants" and "diaper-pants".
"Longitudinal" refers to a direction running substantially perpendicular from a waist edge to an opposing waist edge of the article and generally parallel to the maximum linear dimension of the article.
"Transverse" refers to a direction perpendicular to the longitudinal direction.
"Proximal" and "distal" refer respectively to the position closer or farther relative to the longitudinal center of the article.
"Body-facing" and "garment-facing" refer respectively to the relative location of an element or a surface of an element or group of elements. "Body-facing" implies the element or surface is nearer to the wearer during wear than some other element or surface. "Garment-facing" implies the element or surface is more remote from the wearer during wear than some other element or surface (i.e., element or surface is proximate to the wearer's garments that may be worn over the disposable absorbent article).
"Disposed" refers to an element being located in a particular place or position.
"Joined" refers to configurations whereby an element is directly secured to another element by affixing the element directly to the other element and to configurations whereby an element is indirectly secured to another element by affixing the element to intermediate member(s) which in turn are affixed to the other element.
"Film" refers to a sheet-like material wherein the length and width of the material far exceed the thickness of the material. Typically, films have a thickness of about 0.5 mm or less.

"Water-permeable" and "water-impermeable" refer to the penetrability of materials in the context of the intended usage of disposable absorbent articles. Specifically, the term "water-permeable" refers to a layer or a layered structure having pores, openings, and/or interconnected void spaces that permit liquid water, urine, or synthetic urine to pass through its thickness in the absence of a forcing pressure. Conversely, the term "water-impermeable" refers to a layer or a layered structure through the thickness of which liquid water, urine, or synthetic urine cannot pass in the absence of a forcing pressure (aside from natural forces such as gravity). A layer or a layered structure that is water-impermeable according to this definition may be permeable to water vapor, i.e., may be "vapor-permeable".

"Extendibility" and "extensible" mean that the width or length of the component in a relaxed state can be extended or increased.

"Elasticated" and "elasticized" mean that a component comprises at least a portion made of elastic material.

"Elongatable material", "extensible material", or "stretchable material" are used interchangeably and refer to a material that, upon application of a biasing force, can stretch to an elongated length of at least about 110% of its relaxed, original length (i.e. can stretch to 10 percent more than its original length), without rupture or breakage, and upon release of the applied force, shows little recovery, less than about 20% of its elongation without complete rupture or breakage as measured by EDANA method 20.2-89. In the event such an elongatable material recovers at least 40% of its elongation upon release of the applied force, the elongatable material will be considered to be "elastic" or "elastomeric." For example, an elastic material that has an initial length of 100mm can extend at least to 150mm, and upon removal of the force retracts to a length of at least 130mm (i.e., exhibiting a 40% recovery). In the event the material recovers less than 40% of its elongation upon release of the applied force, the elongatable material will be considered to be "substantially non-elastic" or "substantially non-elastomeric". For example, an elongatable material that has an initial length of 100mm can extend at least to 150mm, and upon removal of the force retracts to a length of at least 145mm (i.e., exhibiting a 10% recovery).

"Radial" means a direction running from the center of a drum toward a drum outer circumferential surface.

"Substrate" means a material which is primarily two-dimensional (i.e. in an XY plane) and whose thickness (in a Z direction) is relatively small (i.e. 1/10 or less) in comparison to its length (in an X direction) and width (in a Y direction). Non-limiting examples of substrates include a web, layer or layers or fibrous materials, nonwovens, films and foils such as polymeric films or metallic foils. These materials may be used alone or may comprise two or more layers laminated together. As such, a web is a substrate.

"Nonwoven" means a material made from continuous (long) filaments (fibers) and/or discontinuous (short) filaments (fibers) by processes such as spunbonding, meltblowing, carding, and the like. nonwovens do not have a woven or knitted filament pattern.

"Machine direction" means the direction of material flow through a process. In addition, relative placement and movement of material can be described as flowing in the machine direction through a process from upstream in the process to downstream in the process.

"Cross machine direction" means a direction that is generally perpendicular to the machine direction.

"Dimension", "Length", "Width", "Pitch", "Interval", "Diameter", "Aspect Ratio", "Angle", and "Area" of the article are all measured in a state wherein the article is extended to the Full Circumference according to the "Whole Article Force Measurement" herein, and utilizing a ruler or a loupe, unless specified otherwise.

"Artwork" refers to a visual presentation to the naked eye, which is provided by printing or otherwise, and having a color. Printing includes various methods and apparatus well known to those skilled in the art such as lithographic, screen printing, flexographic, and gravure ink jet printing techniques.

"Color" or "Colored" as referred to herein includes any primary color except color white, i.e., black, red, blue, violet, orange, yellow, green, and indigo as well as any declination thereof or mixture thereof. The color white is defined as those colors having a L* value of at least 94, an a* value equal to $0 \pm 2$, and a b* value equal to $0 \pm 2$ according to the CIE L* a* b* color system.

DETAILED DESCRIPTION OF THE INVENTION

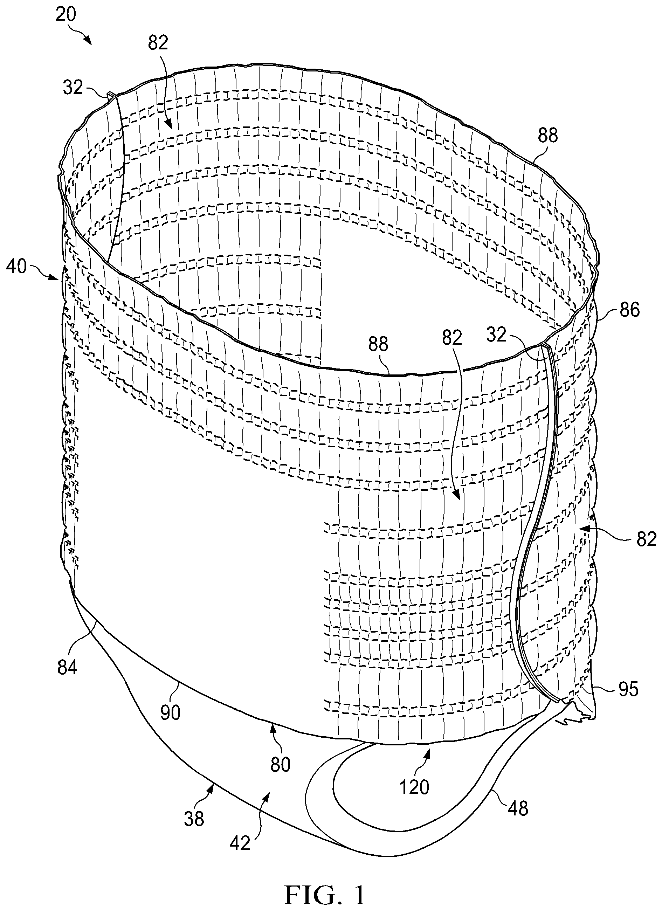

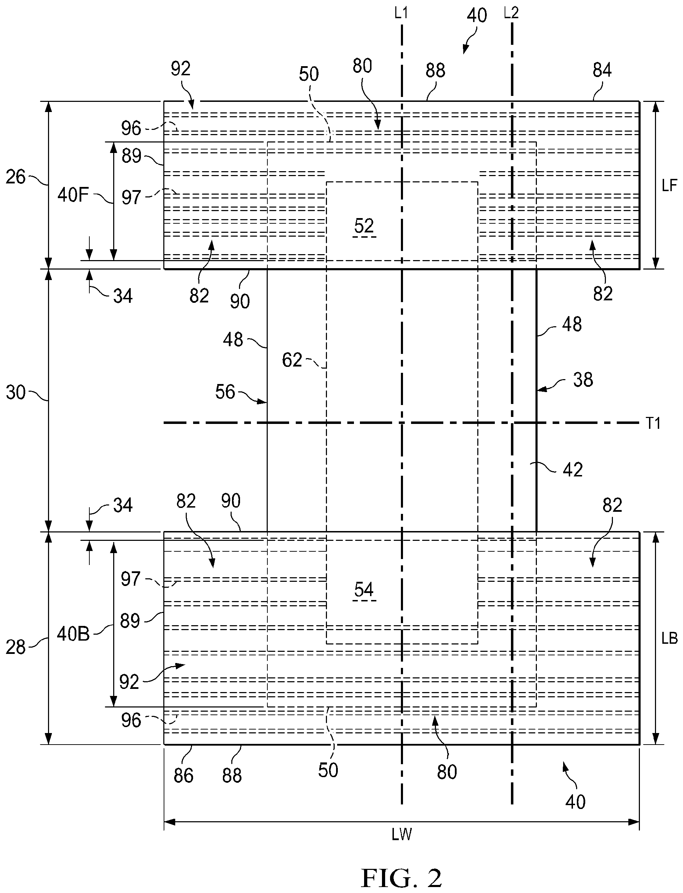

Figure 1 is a perspective view of an embodiment of the wearable article (20) of the present invention and Figure 2 is a schematic plan view of the same article (20) with the seams (32) unjoined and in its flat uncontracted condition showing the garment-facing surface. The wearable article (20) has a longitudinal centerline L1 which also serves as the longitudinal axis, and a transverse centerline T1 which also serves as the transverse axis. The wearable article (20) has a body facing surface, a garment facing surface, a front region (26), a back region (28), a crotch region (30), and seams (32) which join the front region (26) and the back region (28) to form two leg openings and a waist opening. The wearable article (20) comprises a center chassis (38) to cover the crotch region of the wearer, a front belt (84) and a back belt (86) (hereinafter may be referred to as "front and back belts (84, 86)", the front and back belts (84, 86) forming a discrete ring-like elastic belt (40). The front and back belts (84, 86) and the center chassis (38) may jointly define the leg openings.

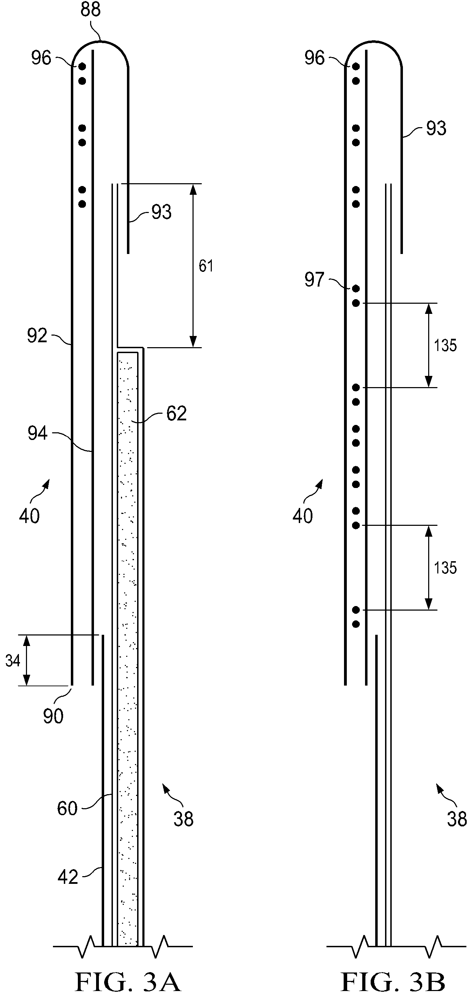

Referring to Figure 3A, the center chassis (38) may comprise a backsheet (60) and an outer cover layer (42) for covering the garment-facing side of the backsheet (60). The backsheet (60) may be a water impermeable film. The outer cover layer (42) may be a nonwoven sheet. The center chassis (38) may contain an absorbent core (62) for absorbing and containing body exudates disposed on the center chassis (38). In the embodiment shown in Figure 2, the center chassis (38) has a generally rectangular shape, left and right longitudinally extending side edges (48) (hereinafter may be referred to as "side edge") and front and back transversely extending end edges (50) (hereinafter may be referred to as "end edge"). The center chassis (38) also has a front waist panel (52) positioned in the front region (26) of the wearable article (20), a back waist panel (54) positioned in the back region (28), and a crotch panel (56) between the front and back waist panels (52, 54) in the crotch region (30). The center of the front belt (84) is joined to a front waist panel (52) of the center chassis (38), the center of the back belt (86) is joined to a back waist panel (54) of the center chassis (38), the front and back belts (84, 86) each having a left side panel and a right side panel (82) where the center chassis (38) does not overlap.

The elastic belt (40) of the article (20) of the present invention acts to dynamically create fitment forces and to distribute the forces dynamically generated during wear. The proximal edge (90) is located closer than the distal edge (88) relative to the crotch panel (56) of the center chassis (38). The front and back belts (84, 86) may be joined with each other only at the side edges (89) at the seams (32) to form a wearable article (20) having a waist opening and two leg openings. Each leg opening may be provided with elasticity around the perimeter of the leg opening by the combination of elasticity from the front belt (84), the back belt (86), and any from the center chassis (38).

The transverse width of the backsheet (60) and the outer cover layer (42) may be the same or may be varied (not shown). For example, the backsheet (60) may have a shorter transverse width compared to that of the outer cover layer (42). By such configuration, the longitudinal side edges (48) of the crotch panel (56), which make part of the leg openings, may have better breathability.

The front belt (84) and back belt (86) are configured to impart elasticity to the belt (40). The front belt (84) and the back belt (86) may each be formed by a laminate comprising a plurality of elastic bodies (96, 97) running in the transverse direction, an inner sheet (94), an outer sheet (92), and an outer sheet fold over (93) wherein the outer sheet fold over (93) is an extension of the outer sheet (92) material formed by folding the outer sheet (92) material at the distal edge (88) of the front and back belts (84, 86); wherein elastic bodies (96, 97) are sandwiched between two of these sheets. The front belt (84) and the back belt (86) may each be made only by elastic bodies (96, 97) the inner sheet (94), the outer sheet (92), and the outer sheet fold over (93). The elastic bodies (96, 97) may extend in the transverse direction to provide a ring like elastic belt (40) when the front belt (84) and the back belt (86) are joined. At least some of the elastic bodies (96, 97) extend in the transverse direction substantially parallel to each other. All of the elastic bodies (96, 97) may extend in the transverse direction substantially parallel to each other. Such an article (20) may be economically made. The front and back belt (84, 86) each may have transversely continuous proximal and distal edges (88, 90), the proximal edge (90) being located closer than the distal edge (88) relative to the longitudinal center of the article (20). The elastic bodies (96, 97) may be disposed in the same or different denier, interval, and force between the front and back, as well as in different longitudinal positions of the belt.

The front and/or back belt (84, 86) may be treated such that certain of the area overlapping the front and/or back waist panel (52, 54) of the center chassis (38) are removed of elasticity. Removal of elasticity from a certain area of the front and/or back waist panel (52, 54) may be advantageous when the center chassis (38) comprises an absorbent core (62), in that elasticity in the front and/or back area overlapping the absorbent core (62) may cause bunching of the absorbent layer or any of the layers in the absorbent core (62) and interfere with close fit of the center chassis (38) to the wearer. Figure 3A is a cross section view of Figure 2 taken along L1 wherein the absorbent core (62) overlaps the elastic belt (40). Figure 3B is a cross section view of Figure 2 taken along L2 wherein the absorbent core (62) does not overlap the elastic belt (40). Herein, those elastic bodies which extend in active elasticity between the side seams (32) are defined waist elastic bodies (96). Referring to Figures 2, 3A, and 3B, the waist elastic bodies (96) extend in active elasticity along the entire transverse dimension of the belt (84, 86). Herein, those elastic bodies which are removed of elasticity over a certain dimension in the transverse direction are defined tummy elastic bodies (97). There may be at least about 4 tummy elastic bodies (97), or at least about 6 tummy elastic bodies (97) per the front or back belt (84, 86). Referring to Figures 2, 3A, and 3B, the tummy elastic bodies (97) may be removed of elasticity along a certain transverse dimension in which each elastic body overlaps with the absorbent core (62). Referring to Figure 2, the entire area where the elastic bodies overlap with the absorbent core (62) may be removed of its elasticity as in the front belt (84). Alternatively, as seen in the back belt (86), the elastic bodies overlapping the absorbent material non-existing region (61) and toward the distal edge (88) of the absorbent core (62) may be disposed in active elasticity for good fit of the center chassis (38). This may be advantageous in preventing leakage. Herein, those elastic bodies which overlap the absorbent core (62) and are not removed of elasticity are defined as waist elastic bodies (96). By removal of elasticity, what is meant is that the particular portion of the elastic body is non-contributory to elasticity of the elastic belt (40).

As a result of removal of elasticity of the tummy elastic bodies (97), there is provided a non-elastic region (22). The non-elastic region (22) is defined by the smallest transverse dimension in which the tummy elastic bodies (97) are removed, and the longitudinal dimension from the proximal edge (90) of the belt to the waist elastic body (96) closest to the proximal edge. The longitudinal dimension of the non-elastic region (22) may be provided as at least about 40mm, or at least about 50mm.

Referring to Figure 2, the transverse width LW of the back belt (86) in the uncontracted condition may be the same as the transverse width of the front belt (84) of the same condition. Such an article (20) may be economically made.

The longitudinal length LB of the back belt (86) between the back distal edge (88) and the back proximal edge (90) along its entire width LW of the back belt (86) may be approximately the same as the longitudinal length LF of the front belt (84) between the front distal edge (88) and the front proximal edge (90). In such configuration, the seams (32) close the front and back belt (84, 86) side edges (89) of the same length for forming the article (20). Such an article (20) may be economically made.

Referring to Figures 1 and 2, the back belt (86) may have a greater longitudinal length LB between the back distal edge (88) and the back proximal edge (90) along its entire width LW of the back belt (86) in the transverse direction than the longitudinal length LF of the front belt (84) between the front distal edge (88) and the front proximal edge (90). In such configuration, when the wearable article (20) is assembled to form the waist opening and the leg openings, the wearable article (20) is folded along the transverse centerline T1 such that the front distal edge (88) is aligned with the back distal edge (88). The front side edge (89) is also aligned with a portion of the back side edge (89). Then the front belt (84) and the back belt (86) are joined at the front and back side edges (89) at the seams (32). The front and back proximal edges (90), however, may not be aligned to one another. The back proximal edge (90) may be disposed longitudinally closer than the front proximal edge (90) relative to the transverse center line T1 such that the proximal portion of the back side panel (82) extends toward the crotch panel (56) of the center chassis (38) beyond the front proximal edge (90). The side edge of the proximal portion of the back side panel (82) may not be joined to anywhere and free from attachment. Thus, the proximal portion of the back side panel (82) provides a buttock cover (95) as in Figure 1.

The elastic belt (40) may be closely associated with the function and quality of the article (20). Thus, materials for forming the elastic belt (40), as well as the elastic profile of the elastic belt (40), are carefully selected by the manufacturer for providing the desirables for the article (20). An undergarment kind of appearance and pleasant tactile sense such as flexibility and cushiony touch may be associated with high quality. Ease of stretch and application, while also maintaining certain force during wear to prevent the article (20) from sagging after loading, may be associated with high function. The balance of the aforementioned quality and function attributes provide the favorable entire usage experience of the article (20) by the user. The user may be the wearer or the caregiver.

The article (20) of the present invention may have a suitable Stretch Circumference Force (N). What is meant by Stretch Circumference Force is the loading force at a certain stretch level which is believed to simulate initial stretch experience felt by the user when inserting hands and stretch opening the article (20). The level of stretch which is believed to be felt by the user when stretch opening the article (20) is represented by the "470mm Stretch Circumference". The dimension of 470mm is selected based on a study by the Applicant whereby the average standing hip circumference (mm) at the height matching the pubic bone of children having a body weight of 6-20kg was 473mm based on data from over 1000 subjects. Namely, according to common habits for wearing a pant article (20), the user would stretch open the elastic belt (40) of the pant article (20) to a circumference more or less matching that of the hip circumference of the body of the wearer. A body weight of 6-20kg matches the recommended body weight of wearers for pant articles (20) of Sizes 3-6 (Sizes M to XXL). The article (20) of the present invention has a Stretch Circumference Force of no greater than about 8.0N, or no greater than about 7.6N. Without being bound by theory, it is believed that the lower the Stretch Circumference Force, the elastic belt (40) may be stretched with less force, thus softer the perception of the elastic belt (40) by the user.

The article (20) of the present invention may have a suitable Fit Circumference Force (N). What is meant by Fit Circumference Force is the unloading force at a certain stretch level which is believed to simulate the force felt by the wearer while wearing the article (20). The level of stretch which is believed to be felt by the wearer while wearing the article (20) is also represented by the 470mm Stretch Circumference. The article (20) of the present invention has a Fit Circumference Force of no less than about 2.0N, or no less than about 2.5N. By having such Fit Circumference Force, the elastic belt (40) provides good fit to prevent sagging and leakage. Without being bound by theory, it is believed that by having a relatively low Stretch Circumference Force of no greater than about 8.0N in combination with a minimum Fit Circumference Force of no less than about 2.0N, an elastic belt (40) having ease of application and a secure yet soft fit may be provided.

The Fit Circumference Force (N) and Stretch Circumference Force (N) are obtained according to the "Whole Article Force Measurement" below.

The tensile stress (N/m) of the front and back elastic belts (84, 86), respectively, may be profiled depending on the zone of the front and back elastic belts (84, 86). By zone, what is meant is a certain longitudinal dimension of the front or back elastic belt (84, 86) comprising at least 2 elastic bodies (96, 97). The tensile stress of a certain zone may be adjusted by one or more of the following methods; 1) elongation rate of the elastic body; 2) density (dtex) of the elastic body; 3) longitudinal interval of multiple elastic bodies; and 4) effective length of elasticity of the elastic body in the transverse direction. By elongation, "0% elongation" is meant the original length of the elastic body. When a portion of an elastic body is removed of its elasticity, the remainder of the intact elastic body capable of imparting elasticity to the elastic belt (40) is defined as the "effective length of elasticity of an elastic body". Without being bound by theory, such profiling of the tensile stress per zone is believed to provide the article (20) of the present invention with a shaped elastic belt (40) that conforms well to a human body, particularly to a lower torso of a child of less than 36 months of age, and therefore provide good fit and comfort to the wearer, without compromise of sagging prevention or leakage prevention. Namely, a certain zone on the front may be subject to high tensile stress such that the article (20) may be anchored against the wearer's trochanter, while leaving more area for the back to accommodate the wearer's buttock. So long as the article (20) is anchored securely at the trochanter, the zone adjacent the leg opening may be provided with significantly less tensile stress, thus disposed of elastic bodies of low density. Soft fit at the front leg opening region may facilitate leg movement during wear.

Certain zones of the belt are disposed of elastic bodies having a density of less than about 500dtex. Elastic bodies having a density of less than about 500dtex are tummy elastic bodies (97), and/or may be disposed adjacent the proximal edge (90) of the elastic belt (40) along the zone adjacent the leg opening, for providing the benefit as explained above. Without being bound by theory, it is believed that elastic bodies of relatively low density impart an easy initial stretch experience when stretch opening the article (20), while maintaining a good fit during wear. Namely, use of such elastic bodies of relatively low density are advantageous in providing a controlled Stretch Circumference Force, while maintaining a certain Fit Circumference Force.

The tummy elastic bodies (97) are disposed on the front and/or back belt (84, 86) in a tummy elastic array comprising a plurality of intervals between the tummy elastic bodies (97), wherein the tummy elastic array comprises a major interval (135) having a longitudinal dimension of at least about 10mm, or at least about 15mm. The remainder intervals of the tummy elastic array may have a smaller longitudinal dimension, such as from about 2mm to about 8mm, or from about 2mm to about 4mm, or from about 5mm to about 8mm, or from about 7mm to about 9mm. The remainder intervals of the tummy elastic array may be even or varied. There may be one or more major intervals (135) per tummy elastic array. The major interval (135) of the tummy elastic array may be provided for separating zones, or for providing a portion of the elastic belt (40) having low tensile stress, or for facilitating process. The tummy elastic array may comprise at least 2, or at least 4, elastic bodies having a density of less than about 500dtex. The tummy elastic array is made only of elastic bodies having a density of less than about 500dtex.

The obtained wearable article (20) of the present invention may provide high quality perception, ease of application, fit, comfort during wear, prevention of sagging, and prevention of leakage.

The outer sheet (92) for forming the elastic belt (40) may have a certain material thickness to provide the lofty undergarment-like appearance and feel, for example, at least about 0.25mm, or at least about 0.3mm. Suitable for the outer sheet (92) of the present invention include: hi-loft nonwoven, air-through carded nonwoven, and spunbond nonwoven made of crimping fiber made through core eccentric bicomponent filament or side by side bicomponent filament, preferably air-through carded nonwoven. Non-limiting examples of materials suitable for the outer sheet (92) include: 20-50gsm air-through carded nonwoven made of less than 15$\mu$m diameter PE/PET bicomponent staple fiber, such as those with a tradename of FJ206 available from Dayuan, Beijing China.

The inner and outer sheets (92, 94) may be the same or different material, and selected to provide characteristics such as breathability, softness, cushiony feel, loftiness, and combinations thereof, depending on the desirables of the resulting article (20). The inner and outer sheets (92, 94) may have the same or different basis weight, stiffness, texture or any combination thereof. The outer sheet (92) may have higher basis weight than the inner sheet (94) for providing the favorable tactile acceptance as discussed above, while controlling cost.

Suitable for the inner sheet (94) of the present invention include: 10-40gsm soft nonwoven, spunbond nonwoven with filament additive slip agent, spun high-loft nonwoven or air-through carded nonwoven, preferably spun high-loft nonwoven.

The material for the outer cover layer (42) may be selected to provide characteristics such as breathability, softness, cushiony feel, loftiness, and combinations thereof, depending on the desirables of the resulting article (20). The outer cover layer (42) may be made of the same material as the outer sheet (92) to provide integral aesthetic and tactile senses for the article (20). By "the same material", what is meant is that the nonwoven has the same type of filament in shape, composition, diameter difference of no more than 2$\mu$m, and basis weight difference of no more than 2gsm.

The inner and outer sheets (92, 94) may be provided with a repetition of openings, such as slits, mesh holes, or apertures. The outer sheet (92) may be provided with apertures. The individual aperture may be in the shape of a circle, oval, or polyhedron, while having an aspect ratio of no more than about 2. The individual aperture may have a minor radius of at least about 0.1mm, or from about 0.1mm to about 0.8mm. What is meant by minor radius herein is the radius of a circle, minor radius of an oval, or one half the shortest dimension of a polyhedron. Apertures of such size and shape may be visible to the naked eye on the garment-facing surface, and thus connote breathability and high quality of the article (20).

Referring to Figures 2 and 3A, the front and back belts (84, 86) are discontinuous with one another in the crotch region (30), and the outer cover layer (42) is the garment-facing surface in the crotch region (30). The outer cover layer (42) may extend only partly in the longitudinal direction of the front waist panel (52) and the back waist panel (54) to leave the distal parts of the front waist panel (52) and the back waist panel (54) free of the outer cover layer (42). Namely, the longitudinal length of the outer cover layer (42) may be longer than the longitudinal length of the crotch panel (56) and shorter than the longitudinal length of the backsheet (60). By such configuration, the distal parts of the front waist panel (52) and the back waist panel 54 are devoid of the outer cover layer (42), providing better breathability to the overall article (20).

Accordingly, looking at the layers of elements between the garment facing surface and the backsheet (60) of the center chassis (38) of Figure 3A, there exists a transitional region (34) disposed on the waist panel (52) where the outer cover layer (42) is present. The longitudinal length of the transitional region (34) may be made as short as possible, for example, less than about 20mm, or less than about 15mm, or less than about 10mm.

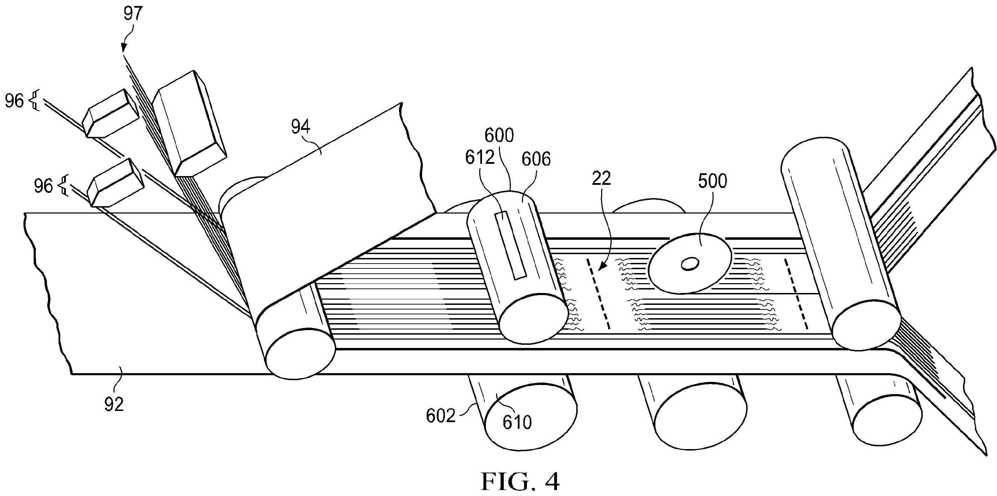

Referring to Figures 3A and 4, for providing attractive artwork for a wearable article (20) in an economical manner, printing may be provided on the garment facing side of the backsheet (60). Alternatively and/or additionally, artwork may be provided by printing on the garment facing surface of the inner sheet (94) or the body facing surface of the outer sheet (92). Whether the artwork is printed on the backsheet (60) or one of the inner or outer sheets (92), the artwork may comprise a main artwork, not shown, attracting the attention of the intended wearer, such as a character, a face, or an object of liking of the intended wearer. The main artwork may be disposed in or overlapping the non-elastic region (22) so that distortion by elasticity of the elastic belt (40) is minimized. As discussed hereinbelow in detail, the non-elastic region (22) may be made, during manufacture, by contacting the non-elastic region (22) with a blade (612) at certain pressure for severing the tummy elastic bodies (97) while not cutting through the inner and outer sheets (92, 94), thus removing elasticity of the tummy elastic bodies (97). The blade (612) may contact the assembly from the inner sheet (94) side, so as to minimize impact to the outer sheet (92) which may be made of softer material and being the garment-facing side. The contact force for such severing method may be controlled so as not to cut through the inner and outer sheets (92, 94), however, may still leave a contact trace, not shown, on the outer sheet (92) at locations which were contacted by the blade (612). The contact trace thus may be seen from the garment facing side. The contact trace may take the appearance of an embossed line which is parallel to the longitudinal axis L1. The position of severing the tummy elastic bodies (97) by the blade (612) may be so configured such that the contact trace does not overlap with the main artwork.

The front belt and the back belt (84, 86) of the present invention each have a Belt Tensile Strength of at least about 17.3 N/cm, or at least about 17.8 N/cm according to the measurements herein. Without being bound by theory, by controlling the Belt Tensile Strength as such, the front belt and the back belt (84, 86) may each have sufficient durability to avoid rupturing for the remainder of the manufacturing method, as well as handling during compression for packaging, and during use.

The present invention is also directed to a method of making the aforementioned ring-like elastic belt (40). More specifically, the present method relates to making the non-elastic region (22) of the front and back elastic belt (84, 86). Referring to Figure 4, the ring-like elastic belt (40) of the present invention is formed by the following steps. First, a continuous outer sheet (92), a continuous inner sheet (94), a plurality of continuous waist elastic bodies (96) in a stretched state, and a plurality of continuous tummy elastic bodies (97) in a stretched state; are advanced in the machine direction; and then the continuous waist and tummy elastic bodies (96, 97) are joined on the inner facing surfaces of the continuous inner and outer sheets (92, 94). The joined assembly is then advanced to a cutting roll system for removing the elasticity of the planned non-elastic regions (22). The treated assembly is then separated into the continuous front belt (84) and the continuous back belt (86). In Figure 4, the depiction after joining of the continuous inner and outer sheets (92, 94) is shown as if the inner sheet (94) is removed and therefore the elastic bodies can be directly observed.

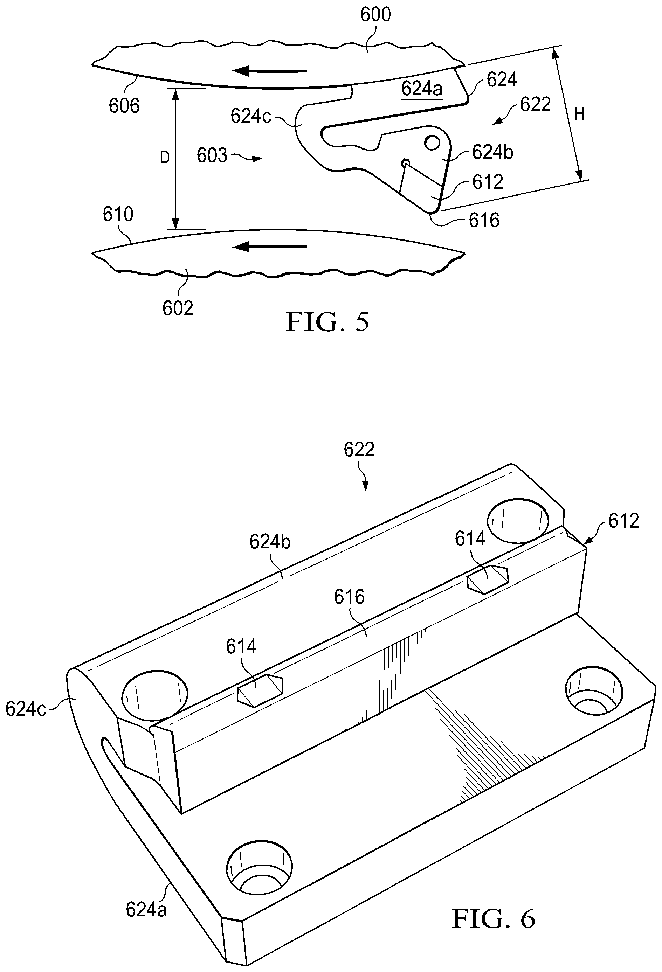

Referring to Figures 4 and 5, the present method comprises forming the non-elastic region (22) by advancing the continuous elastic belt (40) in a machine direction to a cutting roll system for pressure cutting the tummy elastic bodies (97), the cutting roll system comprising an anvil roll (602); and a cutting roll (600) adjacent the anvil roll (602), the cutting roll (600) adapted to rotate about a roll axis perpendicular to the machine direction. The cutting roll (600) comprises a blade (612). Referring to Figure 6, the blade (612) is configured to match the planned non-elastic region (22) of the belt and being perpendicular to the roll axis direction, the blade (612) comprising a blade edge (616) and a recess (614). Referring to Figures 5 and 6, the cutting roll (600) is adjacent to the anvil roll (602) and create a nip (602) defined by a minimum distance D between the outer circumferential surface (606) of the cutting roll (602) and the outer circumferential surface (610) of the anvil roll (602). The blade (612) may extend radially outward from the outer circumferential surface (606) of the cutting roll (600) to the blade edge (616) by a distance H. The distance H of the blade (612) may be greater than the distance D, thereby creating an interference between the blade edge (616) and the outer circumferential surface (610) of the anvil roll (602). The blade (612) may comprise a recess (614) wherein the distance from the outer circumferential surface (606) of the cutting roll (600) to the recess (614) is HR (HR is not shown). Distance HR is smaller than distance H, and may be equal to or smaller than distance D, thereby creating no interference between the recess (614) and the outer circumferential surface (610) of the anvil roll (602). The cutting roll (600) may include a blade assembly (622) having the blade (612) connected with a support member (624). The support member (624) may include a base portion (624a), a mounting portion (624b) and a flexible portion (624c). The flexible portion (624c) may flex and/or bend to allow the blade (612) to deflect when moving through the nip

(603). The recess (614) is provided so as not to engage with the outer circumferential surface (610) of the anvil roll (602), thus providing substantially no pressure against the elastic belt (40) assembly. The recess (614) is configured to match the major interval (135) of the tummy elastic array, such that the continuous assembly in the major interval (135) is not unnecessarily pressed with the blade (612). Such controlled contact between the blade (612) and the assembly is advantageous in effectively severing the tummy elastic bodies (97), while alleviating damage to the inner and/or outer sheet (92, 94) in the major interval (135) where there is no tummy elastic body (97) to receive the force. The contact force between the blade edge (616) and the anvil roll (602) may be no more than about 600N/mm, for alleviating damage to the inner and/or outer sheet (92, 94). The contact force between the blade edge (616) and the anvil roll (602) may be at least about 300N/mm, to ensure severing of the tummy elastic bodies (97). By damage to the inner and/or outer sheet (92, 94), what is meant is a separation of at least 5mm in the longitudinal direction. Deformations such as contact traces which do not significantly decrease the tensile strength of the belt are not considered a damage.

For effecting severing of the tummy elastic bodies (97) in the intended transverse dimension, the tummy elastic bodies (97) in the non-elastic region (22) are either provided with no joining, or may be joined to the inner and outer sheets (92, 94) at a strength significantly weaker than the elastic region. Prior to joining the tummy elastic bodies (97), the non-elastic region (22) may be continuously applied in both the longitudinal direction and the transverse direction with a hot-melt adhesive agent on the surface of one of the outer sheet (92) and the inner sheet (94), wherein the hot-melt adhesive is applied in an amount of from about 1.5 to about 3 gsm.

Referring to Figure 4, the thus obtained assembly comprising: the lamination of the continuous inner and outer sheets (92, 94), the continuous waist elastic bodies (96) and continuous tummy elastic bodies (97); comprises repetition of an elastic region and a non-elastic region (22). The assembly may be divided by a slitter (500) in the machine direction to form 2 continuous parts, which are further processed to provide continuous front and back belts (84, 86). The dividing may be provided before or after making of the non-elastic region (22).

Components of the wearable article described in this specification can at least partially be comprised of bio-sourced content as described in US 2007/0219521A1 Hird et al published on September 20, 2007, US 2011/0139658A1 Hird et al published on June 16, 2011, US 2011/0139657A1 Hird et al published on June 16, 2011, US 2011/0152812A1 Hird et al published on June 23, 2011, US 2011/0139662A1 Hird et al published on June 16, 2011, and US 2011/0139659A1 Hird et al published on June 16, 2011. These components include, but are not limited to, topsheet nonwovens, backsheet films, backsheet nonwovens, belt nonwovens, and parts constructing the absorbent core such as barrier leg cuff nonwovens, core wrap substrates, acquisition layers, and adhesives.

In at least one embodiment, a wearable article component comprises a bio-based content value from about 10% to about 100% using ASTM D6866-10, method B, or from about 25% to about 75%, or from about 50% to about 60% using ASTM D6866-10, method B.

## 1. Belt Tensile Strength

The Belt Tensile Strength may be measured using an Electronic Tensile Tester with a computer interface such as the MTS Criterion C42 running TestWorks 4 Software (available from MTS SYSTEMS (CHINA) CO., LTD) or equivalent instrument. A load cell is chosen so that force results for the samples tested will be between 10 and 90% of capacity of the load cell. The instrument is calibrated according to the manufacturer's instructions. All testing is performed in a room maintained at 23 $\pm$ 2 °C and 50 $\pm$ 5 % relative humidity. The instrument is equipped with standard upper and lower clamps having single line contact grips at least as wide as the test specimen.

To obtain test specimens, the sample article is opened at both side seams in a manner such that the front and back belts (84, 86) are peeled away from each other without removing the side seam area, and further removed from the center chassis. For each belt, the contact trace is identified. Taking the contact trace as the center line, 50mm each of the transverse dimension is measured. Further, 40mm of the longitudinal dimension is measured from the proximal edge (90). The thus obtained 40mm by 100mm rectangle is cut out using scissors to obtain a specimen. Specimen are pre-conditioned at 23 °C $\pm$ 2 C° and 50% $\pm$ 5% relative humidity for two hours prior to testing.

The instrument is set up as follows. Initial Gauge Length is set at 30mm long between the center of the upper clamp to the center of the lower clamp. The crosshead speed is set at 254mm/min. The data acquisition rate is set at 50Hz. The load is reset to zero. The upper and lower clamps as well as the line of contact trace are set to be parallel with each other and to have a load lower than 0.05N. The test is started and the specimen is extended until it is completely separated into two pieces. The peak load force is recorded during the test.

Five specimen are measured and the peak load forces are recorded. The tensile strength for each specimen is calculated by the average peak load force (N) divided by 5cm of the longitudinal length (cm) and reported to the nearest 0.1 N/cm.

## 2. Whole Article Force Measurement

Force is measured using an Electronic Tensile Tester with a computer interface such as the MTS Criterion C42 running TestWorks 4 Software (available from MTS SYSTEMS (CHINA) CO., LTD) or equivalent instrument. A load cell is selected so that force results for the samples tested will be between 10 and 90% of capacity of the load cell used. The instrument is calibrated according to the manufacturer's instructions. All testing is performed in a room maintained at 23 $\pm$ 2 °C and 50 $\pm$ 5 % relative humidity.

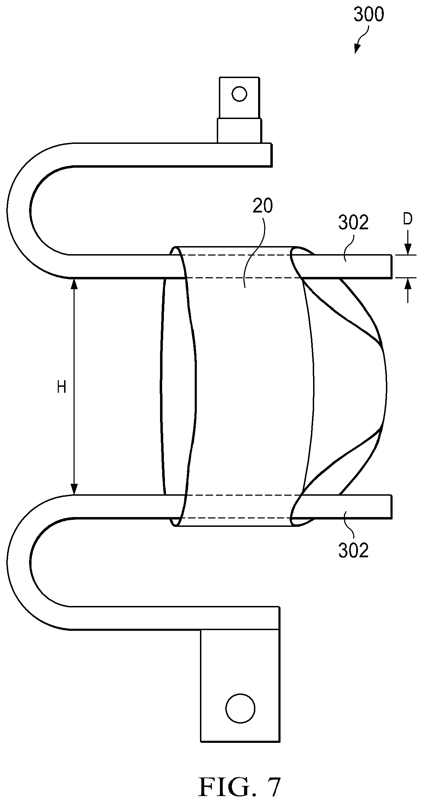

The tensile tester is fitted with hanger-type sample holding fixtures (300) as shown in Figure 7. Each fixture comprises a rigid linear rubber-coated horizontal bar section (302) to prevent sample slippage during testing. The outer bar diameter (including the rubber coating) of the horizontal bar sections is 10.0 mm. The central axes of the horizontal bar sections (302) are configured to remain parallel and in the same vertical plane throughout the test procedure. The gauge circumference is determined by the following equation:

$$\text{Gauge Circumference} = 2 \times (H + D + \pi D/2)$$

where H is the vertical gap between the horizontal bar sections (302), and D is the outer diameter of the bar.

The instrument is set up to go through the following steps:

| | |
|---|---|
| Crosshead Speed | 254.0mm/min |
| Final Load Point | 19.6 N |
| Hold Time | 0 |
| Number of Cycles | 1 |
| Data Acquisition Rate | 50Hz |

A sample article (20) is inserted onto the upper horizontal bar section (302) so that the bar passes through the waist opening and one leg opening of the article (20). The crosshead is raised until the specimen hangs above the lower bar and does not touch lower bar (302). The load cell is tared and the crosshead is lowered to enable the lower bar (302) to be inserted through the waist opening and other leg opening without stretching the article (20). The article (20) is adjusted so that the longitudinal centerline L1 of the article (20) is in a horizontal plane halfway between the upper and lower bars (302). The center of the side portion in contact with the bar (302) is situated on the same vertical axis as the instrument load cell. The crosshead is raised slowly while the article (20) is held in place by hand as necessary until the force is between 0.05 and 0.1N, while taking care not to add any unnecessary force. The gauge circumference at this point is the Initial Gauge Circumference. The test is initiated and the crosshead moves up at a speed of 254 mm/min until a force of 19.6N is attained, then the crosshead immediately returns to the Initial Gauge Circumference at the same speed. The maximum circumference at 19.6N during the extension segment of the test is recorded.

The Stretch Circumference Force is defined as the force at 470mm Stretch Circumference during the load (extension) segment of the test. The Fit Circumference Force is defined as the force at 470mm Stretch Circumference during the unload (contraction) segment of the test.

Five samples are analyzed and their average Stretch Circumference Force and average Fit Circumference Force are calculated and reported to the nearest 0.01 N, respectively.

EXAMPLES

Examples 1-2 and A-B having the structure of a pant type wearable article (20) are obtained and subject to measurements as described above. For all of these Examples, the back belt has 2 major intervals and thus potentially may have lower Belt Tensile Strength compared to the front belt. Thus, for all Examples, measurement was only provided to the back belt.

Example 1: A Size 4 belt-type pant article (20) having the configuration of Figs 1 and 2 with the outer sheet (92) made by tradename FJ206 available from Dayuan, Beijing China (20gsm air-through carded nonwoven with 15μm diameter PE/PET bicomponent fiber) and the inner sheet (94) made by tradename HY15015-MALAYSIA-V2 available from Fibertex (15gsm PP spunbond nonwoven), and manufactured according to the methods of the present invention.

Example 2: A Size 4 belt-type pant article (20) having the same configuration as Example 1 except the outer sheet (92) made by dual 17gsm PE/PP bicomponent spunbond nonwoven available from Fibertex, the inner sheet (94) made by tradename HY15015-MALAYSIA-V2 available from Fibertex (15gsm PP spunbond nonwoven), and manufactured according to the methods of the present invention.

Example A: Pampers Hada Ichiban Pant available in the PRC with Lot No. 20190128 made of the same material as Example 1, however not manufactured according to the methods of the present invention.

Example B: Pampers Super Thin and Dry Pant available in the PRC with Lot No. 20181022 made of the same material as Example 2, however not manufactured according to the methods of the present invention.

Table 1

| Example | Belt Tensile Strength (N/cm) | Stretch Circumference Force (N) | Fit Circumference Force (N) |
|---|---|---|---|
| 1 | 17.8 | 7.6 | 3.9 |
| 2 | 19.6 | 6.5 | 3.1 |
| A | 12.5 | 7.6 | 3.9 |
| B | 16.2 | 6.5 | 3.1 |

Examples 1 and 2 according to the present invention had a higher Belt Tensile Strength which provides an article having sufficient durability to avoid rupturing for the entire manufacturing process, as well as during use. Examples 1 and 2 could be manufactured at approximately the same reliability level and approximately the same speed as manufacturing Examples A and B.

The dimensions and values disclosed herein are not to be understood as being strictly limited to the exact numerical values recited. Instead, unless otherwise specified, each such dimension is intended to mean both the recited value and a functionally equivalent range surrounding that value. For example, a dimension disclosed as "40 mm" is intended to mean "about 40 mm." Further, every numerical range given throughout this specification includes every narrower numerical range that falls within such broader numerical range.

While particular embodiments of the present invention have been illustrated and described, it would be obvious to those skilled in the art that various other changes and modifications can be made without departing from the scope of the invention. It is therefore intended to cover in the appended claims all such changes and modifications that are within the scope of the appended claimed.

**Claims**

1. A ring-like elastic belt (40) having a longitudinal direction and a transverse direction comprising:

    a front belt (84) and a back belt (86), each of the front belt (84) and the back belt (86) being an elastic laminate comprising an outer sheet (92), an inner sheet (94), a plurality of waist elastic bodies (96) joined between the outer sheet (92) and the inner sheet (94) and extending transversely substantially parallel to each other, and a plurality of tummy elastic bodies (97) joined between the outer sheet (92) and the inner sheet (94) and extending transversely substantially parallel to each other,
    each of the front belt (84) and the back belt (86) further comprising a non-elastic region (22) wherein each of the tummy elastic bodies (97) are removed of elasticity over a certain dimension in the transverse direction,
    wherein the outer sheet (92) is a nonwoven fabric and the inner sheet (94) is a nonwoven fabric;
    wherein the tummy elastic bodies (97) are disposed in a tummy elastic array comprising a plurality of intervals between the tummy elastic bodies (97), wherein the tummy elastic array comprises a major interval (135) having a longitudinal dimension of at least about 10mm, preferably at least about 15mm;
    wherein the front belt and the back belt each have a Belt Tensile Strength of at least about 17.3 N/cm, preferably at least about 17.8N/cm, according to the measurements herein;
    wherein the tummy elastic bodies (97) have a density of less than about 500dtex.

2. The belt of Claim 1 wherein the outer sheet (92) is a nonwoven fabric made of air-through carded fibers.

3. The belt of any of the preceding claims wherein at least one of the outer sheet (92) and the inner sheet (94) has a repetition of openings.

4. The belt of Claim 3 wherein the outer sheet (92) comprises openings which are apertures having an aspect ratio of no more than about 2 and a minor radius of at least about 0.1mm, preferably from about 0.1mm to about 0.8mm.

5. The belt of any of the preceding claims wherein the outer sheet (92) and the inner sheet (94) are nonwoven materials having difference in at least one of basis weight, stiffness, and texture.

6. A wearable article (20) comprising the ring-like elastic belt (40) of any of the preceding claims and a center chassis (38) connecting the front belt (84) and the back belt (86), wherein the article has a Stretch Circumference Force of less than about 8.0N according to the measurements herein.

7. The article of Claim 6 wherein the article has a Fit Circumference Force of at no less than about 2.0N according to the measurements herein.

8. The article of Claim 6 or 7 wherein the non-elastic region (22) comprises a contact trace resulting from removing elasticity of the tummy elastic bodies (97), wherein the non-elastic region (22) comprises a main artwork observable from the garment-facing side of the belt, wherein the main artwork is disposed so that it does not overlap with the contact trace.

9. A method of manufacturing the ring-like elastic belt (40) of any of the preceding claims wherein the non-elastic region (22) is formed by advancing the continuous elastic belt (40) in a machine direction to a cutting roll system for pressure cutting the tummy elastic bodies (97), the cutting roll system comprising:

   an anvil roll (602); and
   a cutting roll (600) adjacent the anvil roll (602), the cutting roll (600) adapted to rotate about a roll axis perpendicular to the machine direction, and wherein the cutting roll (600) comprises a blade (612), the blade (612) configured to match the non-elastic region (22) of the belt and being perpendicular to the roll axis direction, the blade (612) comprising a recess (614), the recess (614) configured to match the major interval (135); wherein the tummy elastic bodies (97) have a density of less than about 500dtex.

10. The method of Claim 9 wherein the non-elastic region (22) is formed by continuously applying in both the longitudinal direction and the transverse direction a hot-melt adhesive agent, on the surface of one of the outer sheet (92) and the inner sheet (94) to which the tummy elastic bodies (97) are to be joined, in an amount of from about 1.5 to about 3 gsm, followed by joining the tummy elastic bodies (97).

**Patentansprüche**

1. Ringartiger Gummibandbund (40), der eine Längsrichtung und eine Querrichtung aufweist, umfassend:

   einen vorderseitigen Bund (84) und einen rückseitigen Bund (86), wobei jeder des vorderseitigen Bunds (84) und des rückseitigen Bunds (86) ein Gummibandlaminat ist, umfassend eine äußere Lage (92), eine innere Lage (94), eine Vielzahl von Gummibandtaillenkörpern (96), die zwischen der äußeren Lage (92) und der inneren Lage (94) gefaltet sind und sich quer im Wesentlichen parallel zueinander erstrecken, und eine Vielzahl von Gummibandbauchkörpern (97), die zwischen der äußeren Lage (92) und der inneren Lage (94) gefaltet sind und sich quer im Wesentlichen parallel zueinander erstrecken,
   jeder des vorderseitigen Bunds (84) und des rückseitigen Bunds (86) ferner umfassend einen nichtelastischen Bereich (22), wobei jedem der Gummibandbauchkörper (97) in der Querrichtung über eine bestimmte Abmessung seine Elastizität entzogen ist,
   wobei die äußere Lage (92) ein Vliesstoff ist und die innere Lage (94) ein Vliesstoff ist;
   wobei die Gummibandbauchkörper (97) in einer
   Gummibandbauchanordnung angeordnet sind, umfassend eine Vielzahl von Intervallen zwischen den Gummibandbauchkörpern (97), wobei die Gummibandbauchanordnung ein Hauptintervall (135) umfasst, das eine Längsabmessung von wenigstens etwa 10 mm, vorzugsweise wenigstens etwa 15 mm, aufweist;
   wobei der vorderseitige Bund und der rückseitige Bund gemäß den Messungen hierin jeweils eine Bundzugfestigkeit von wenigstens etwa 17,3 N/cm, vorzugsweise wenigstens etwa 17,8 N/cm, aufweisen;
   wobei die Gummibandbauchkörper (97) eine Dichte von weniger als etwa 500 dtex aufweisen.

2. Bund nach Anspruch 1, wobei die äußere Lage (92) ein Vliesstoff ist, der aus luftkardierten Fasern hergestellt ist.

3. Bund nach einem der vorstehenden Ansprüche, wobei wenigstens eine der äußeren Lage (92) und der inneren Lage (94) eine Wiederholung von Öffnungen aufweist.

4. Bund nach Anspruch 3, wobei die äußere Lage (92) Öffnungen umfasst, die Durchlässe sind, die ein Seitenverhältnis von nicht mehr als etwa 2 und einem Nebenradius von wenigstens etwa 0,1 mm, vorzugsweise von etwa 0,1 mm bis etwa 0,8 mm, aufweisen.

5. Bund nach einem der vorstehenden Ansprüche, wobei die äußere Lage (92) und die innere Lage (94) Vliesmaterialien sind, die eine Differenz in wenigstens einem von Flächengewicht, Steifigkeit und Textur aufweisen.

6. Tragbarer Artikel (20), umfassend den ringartigen elastischen Bund (40) nach einem der vorstehenden Ansprüche und eine zentrale Grundeinheit (38), die den vorderseitigen Bund (84) und den rückseitigen Bund (86) verbindet, wobei der Artikel gemäß den Messungen hierin eine Dehnungsumfangswiderstandskraft von weniger als etwa 8,0 N aufweist.

7. Artikel nach Anspruch 6, wobei der Artikel gemäß den Messungen hierin eine Passumfangswiderstandskraft von nicht weniger als etwa 2,0 N aufweist.

8. Artikel nach Anspruch 6 oder 7, wobei der nichtelastische Bereich (22) eine Kontaktspur umfasst, die aus dem Entfernen der Elastizität der Gummibandbauchkörper (97) resultiert, wobei der nichtelastische Bereich (22) ein Hauptbild umfasst, das von der zum Kleidungsstück zeigenden Seite des Bunds aus sichtbar ist, wobei das Hauptbild angeordnet ist, sodass es sich nicht mit der Kontaktspur überschneidet.

9. Verfahren zum Herstellen des ringartigen elastischen Bunds (40) nach einem der vorstehenden Ansprüche, wobei der nichtelastische Bereich (22) ausgebildet wird, indem der ununterbrochene elastische Bund (40) in einer Maschinenlaufrichtung zu einem Schneidwalzensystem vorgeschoben wird, um die Gummibandbauchkörper (97) unter Druck zu schneiden, das Schneidwalzensystem umfassend:

   eine Ambosswalze (602); und
   eine Schneidwalze (600) angrenzend an die Ambosswalze (602), wobei die Schneidwalze (600) konzipiert ist, um sich um eine Walzenachse senkrecht zu der Maschinenlaufrichtung zu drehen, und wobei die Schneidwalze (600) eine Klinge (612) umfasst, wobei die Klinge (612) konfiguriert ist, um zu dem nichtelastischen Bereich (22) des Bunds zu passen und senkrecht zu der Walzenachsenrichtung zu stehen, die Klinge (612) umfassen eine Aussparung (614), wobei die Aussparung (614) konfiguriert ist, um zu dem Hauptintervall (135) zu passen; wobei die Gummibandbauchkörper (97) eine Dichte von weniger als etwa 500 dtex aufweisen.

10. Verfahren nach Anspruch 9, wobei der nichtelastische Bereich (22) durch ununterbrochenes Auftragen sowohl in der Längsrichtung als auch in der Querrichtung eines Heißklebemittels auf die Oberfläche einer der äußeren Lage (92) oder der inneren Lage (94), mit der die Gummibandbauchkörper (97) gefaltet werden sollen, in einer Menge von etwa 1,5 bis etwa 3 g/m$^2$ und anschließendes Falten der Gummibandbauchkörper (97) ausgebildet wird.

**Revendications**

1. Ceinture élastique annulaire (40) ayant une direction longitudinale et une direction transversale comprenant :

   une ceinture avant (84) et une ceinture arrière (86), chacune de la ceinture avant (84) et de la ceinture arrière (86) étant un laminé élastique comprenant une feuille externe (92), une feuille interne (94), une pluralité de corps élastiques de taille (96) assemblés entre la feuille externe (92) et la feuille interne (94) et s'étendant transversalement de manière sensiblement parallèle les uns aux autres, et une pluralité de corps élastiques de ventre (97) assemblés entre la feuille externe (92) et la feuille interne (94) et s'étendant transversalement de manière sensiblement parallèle les uns aux autres,
   chacune de la ceinture avant (84) et de la ceinture arrière (86) comprenant en outre une zone non élastique (22) dans laquelle chacun des corps élastiques de ventre (97) est dépourvu d'élasticité sur une certaine dimension dans la direction transversale,
   dans laquelle la feuille externe (92) est un non-tissé et la feuille interne (94) est un non-tissé ;
   dans laquelle les corps élastiques de ventre (97) sont disposés dans un réseau élastique de ventre comprenant une pluralité d'intervalles entre les corps élastiques de ventre (97), dans lequel le réseau élastique de ventre comprend un intervalle principal (135) ayant une dimension longitudinale d'au moins environ 10 mm, de préférence d'au moins environ 15 mm ;
   dans laquelle la ceinture avant et la ceinture arrière ont chacune une résistance à la traction de ceinture d'au moins environ 17,3 N/cm, de préférence d'au moins environ 17,8 N/cm, selon les mesures dans la description ;
   dans laquelle les corps élastiques de ventre (97) ont une densité inférieure à environ 500 dtex.

2. Ceinture selon la revendication 1, dans laquelle la feuille externe (92) est un non-tissé constitué de fibres cardées à passage d'air.

3. Ceinture selon l'une quelconque des revendications précédentes, dans laquelle au moins l'une parmi la feuille externe (92) et la feuille interne (94) a une répétition d'ouvertures.

4.  Ceinture selon la revendication 3 dans laquelle la feuille externe (92) comprend des ouvertures qui sont des ouvertures ayant un rapport d'aspect ne dépassant pas environ 2 et un rayon mineur d'au moins environ 0,1 mm, de préférence d'environ 0,1 mm à environ 0,8 mm.

5.  Ceinture selon l'une quelconque des revendications précédentes, dans laquelle la feuille externe (92) et la feuille interne (94) sont des matériaux non tissés présentant une différence dans au moins l'une parmi une masse surfacique, une rigidité et une texture.

6.  Article portable (20) comprenant la ceinture élastique annulaire (40) selon l'une quelconque des revendications précédentes et un châssis central (38) reliant la ceinture avant (84) et la ceinture arrière (86), dans lequel l'article a une force d'étirement de circonférence inférieure à environ 8,0 N selon les mesures dans la description.

7.  Article selon la revendication 6, dans lequel l'article a une force de circonférence d'ajustement non inférieure à environ 2,0 N selon les mesures dans la description.

8.  Article selon la revendication 6 ou 7, dans lequel la région non élastique (22) comprend une trace de contact résultant de l'élimination de l'élasticité des corps élastiques de ventre (97), dans lequel la région non élastique (22) comprend un dessin principal observable depuis le côté de la ceinture faisant face au vêtement, dans lequel le dessin principal est disposé de manière à ne pas se superposer à la trace de contact.

9.  Procédé de fabrication de la ceinture élastique annulaire (40) selon l'une quelconque des revendications précédentes, dans lequel la région non élastique (22) est formée en faisant avancer la ceinture élastique continue (40) dans une direction de machine vers un système de rouleau de coupe pour couper sous pression les corps élastiques de ventre (97), le système de rouleau de coupe comprenant :

    un contre-rouleau (602) ; et
    un rouleau de coupe (600) adjacent au contre-rouleau (602), le rouleau de coupe (600) étant adapté pour tourner autour d'un axe de rouleau perpendiculaire à la direction de machine, et dans lequel le rouleau de coupe (600) comprend une lame (612), la lame (612) étant conçue pour correspondre à la région non élastique (22) de la ceinture et étant perpendiculaire à la direction d'axe de rouleau, la lame (612) comprenant un renfoncement (614), le renfoncement (614) étant conçu pour correspondre à l'intervalle principal (135) ;
    dans lequel les corps élastiques de ventre (97) ont une densité inférieure à environ 500 dtex.

10. Procédé selon la revendication 9, dans lequel la région non élastique (22) est formée par l'application continue, à la fois dans la direction longitudinale et dans la direction transversale, d'un agent adhésif thermofusible sur la surface de l'une de la feuille externe (92) et de la feuille interne (94) auxquelles les corps élastiques de ventre (97) doivent être assemblés, dans une quantité d'environ 1,5 à environ 3 g/m$^2$, suivie de l'assemblage des corps élastiques de ventre (97).

FIG. 1

FIG. 2

FIG. 3A          FIG. 3B

FIG. 4

EP 4 041 170 B1

FIG. 5

FIG. 6

FIG. 7

**REFERENCES CITED IN THE DESCRIPTION**

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 7867529 B
- US 9039855 B
- US 9050213 B
- US 9028632 B
- US 8440043 B
- US 9364965 B
- US 9738002 B
- US 2018221218 A1
- WO 2018152833 A1
- WO 2017133031 A1
- WO 2016029374 A1
- US 2013261589 A1
- WO 2011087503 A
- US 20070219521 A1, Hird
- US 20110139658 A1, Hird
- US 20110139657 A1, Hird
- US 20110152812 A1, Hird
- US 20110139662 A1, Hird
- US 20110139659 A1, Hird